# EUROPEAN PATENT SPECIFICATION

(11) **EP 2 648 543 B1**
(45) Date of publication and mention of the grant of the patent: **21.10.2015**
(21) Application number: 10788208.6
(22) Date of filing: 08.12.2010
(51) Int. Cl.: A23L 1/10, A23L 1/105, A23L 1/308, C12P 19/14, C12P 19/24

(54) **FOOD INGREDIENT COMPRISING HYDROLYZED WHOLE GRAIN**
NAHRUNGSMITTELBESTANDTEIL MIT HYDROLYSIERTEM VOLLKORN
INGRÉDIENT ALIMENTAIRE COMPRENANT DES CÉRÉALES ENTIÈRES HYDROLYSÉES

(43) Date of publication of application: 16.10.2013
(73) Proprietor: Nestec S.A., 1800 Vevey (CH)
(72) Inventor: SCHAFFER-LEQUART, Christelle, CH-1083 Mezieres (CH); ROGER, Olivier, CH-1510 Moudon (CH); WAVREILLE, Anne-Sophie, CH-1004 Lausanne (CH); KUNETZ, Christine, Frances, Twinsburg OH 44087 (US); HOWELL, Scott, John, Macedonia OH 44056 (US)
(74) Representative: Mollet, Beat Max
(86) International application number: PCT/US2010/059487
(87) International publication number: WO 2012/078149

(56) References cited:
- EP-A1- 0 231 729
- EP-A1- 0 312 220
- WO-A1-2010/023351
- US-A- 4 282 319
- US-A- 4 656 040
- US-A- 4 710 386
- US-A- 4 894 242
- US-A- 4 996 063
- US-A- 6 017 569

## Description

### TECHNICAL FIELD OF THE INVENTION

The present invention relates to a food ingredient supplemented with whole grain. In particular the present invention relates to food ingredients which are supplemented with hydrolysed whole grain, where neither taste nor viscosity nor organoleptic properties of the food ingredients have been compromised.

### BACKGROUND OF THE INVENTION

There is now extensive evidence emerging mainly from epidemiological studies that a daily intake of three servings of whole grain products, i.e. 48 g of whole grain, is positively associated with decreased risk of cardiovascular diseases, increased insulin sensitivity and decreased risk of type 2 diabetes onset, obesity (mainly visceral obesity) and digestive system cancers. These health benefits of the whole grains are reported to be due to the synergistic role of the dietary fibers and other components, such as vitamins, minerals and bioactive phytochemicals.

The regulatory authorities in Sweden, the US and the UK have already approved specific heart health claims based on the available scientific substantiation.

Food products comprising dietary fibers are also growing in popularity with consumers, not just because whole grain consumption is now included in some national dietary recommendations but also because whole grain products are considered wholesome and natural. Recommendations for whole grain consumption have been set up by government authorities and expert groups to encourage consumers to eat whole grains. For instance, in the U.S.A, recommendations are to consume 45-80 g of whole grain per day. However, data provided by national dietary surveys in the United Kingdom, the U.S.A. and China show that whole grain consumption varies between 0 and 30 g whole grains per day.

The lack of whole grain products offered on the shelves and the poor organoleptic properties of the available whole grain products are generally identified as barriers for whole grain consumption and restrict the amount of whole grain to be added to e.g. a food ingredient, because, when increased amounts of whole grain are added the physical and organoleptic properties of the food ingredient may change dramatically.

Whole grains are also a recognised source of dietary fibers, phytonutrients, antioxidants, vitamins and minerals. According to the definition given by the American Association of Cereal Chemists (AACC), whole grains, and food made from whole grains, consist of the entire grain seed. The entire grain seed comprises the germ, the endosperm and the bran. It is usually referred to as the kernel.

Moreover, in recent years, consumers have increased attention to the label of food products, e.g. composite food products comprising food ingredients, and they expect manufactured food products to be as natural and healthy as possible. Therefore, it is desirable to develop food and drink processing technologies and food and drink products that limit the use of non-natural food additives, even when such non-natural food additives have been fully cleared by health or food safety authorities.

Given the health benefits of whole grain cereal, it is desirable to provide a whole grain ingredient having as much intact dietary fibers as possible. Food ingredients are a good vehicle for delivering whole grain when forming part of a composite food product. To increase the whole grain content of a composite food product or a serving, it is of course possible to increase the serving size. But this is not desirable as it results in a greater calorie intake. Another difficulty in just increasing the whole grain content of the product is that it usually impacts on physical properties such as the taste, texture and the overall appearance of the food ingredients (organoleptic parameters), as well as its processability.

The consumer is not willing to compromise on the organoleptic properties, in order to increase his daily whole grain intake. Taste, texture and overall appearance are such organoleptic properties.

Obviously, industrial line efficiency is a mandatory requirement in the food industry. This includes handling and processing of raw materials, forming of the composite food product comprising the food ingredients, packaging and later storing, in warehouses, on the shelf or at home.

US 4,282,319 relates to a process for the preparation of hydrolyzed products from whole grain, and such derived products. The process includes an enzymatic treatment in an aqueous medium with a protease and an amylase. The obtained product may be added to different types of products. US 4,282,319 describes a complete degradation of the proteins present in the whole grain.

US 5,686,123 discloses a cereal suspension generated by treatment with both an alpha-amylase and a beta-amylase both specifically generating maltose units and have no glucanase effect.

Thus, it is an object of the present invention to provide food ingredients to be used in a composite food product that are rich in whole grains and in dietary fibers, while maintaining a low calorie intake, that provide an excellent consumption experience to the consumer, and that may be easily industrialised at a reasonable cost without compromising the organoleptic parameters.

### SUMMARY OF THE INVENTION

Accordingly, in a first aspect the invention relates to a food ingredient comprising:
- a savoury flavour component;
- a hydrolyzed whole grain composition; and
- an alpha-amylase or fragment thereof, which alpha-amylase or fragment thereof shows no hydrolytic activity towards dietary fibers when in the active state;
   wherein the hydrolyzed whole grain composition has a substantially intact beta-glucan structure relative to the starting material and/or a substantially intact arabinoxylan structure relative to the starting material.

Another aspect of the present invention relates to a process for preparing a food ingredient according to the present invention, said process comprising:
1) preparing a hydrolyzed whole grain composition, comprising the steps of:
   a) contacting a whole grain component with an enzyme composition in water, the enzyme composition comprising at least one alpha-amylase, said enzyme composition showing no hydrolytic activity towards dietary fibers,
   b) allowing the enzyme composition to react with the whole grain component, to provide a whole grain hydrolysate,
   c) providing the hydrolyzed whole grain composition by inactivating said enzymes when said hydrolysate has reached a viscosity comprised between 50 and 12000 mPa.s measured at 65°C,
2) providing the food ingredient by mixing the hydrolyzed whole grain composition with a premix comprising a savoury flavour component.

A further aspect of the invention relates to a composite food product comprising a food ingredient according to the invention.

### BRIEF DESCRIPTION OF THE DRAWINGS

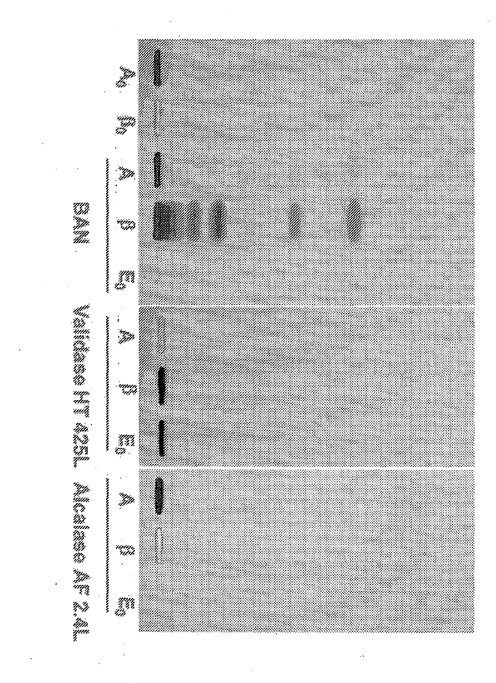

Figure 1 shows a thin layer chromatography analysis of various enzymes put in contact with dietary fibres. The legend for the different tracks is the following:
   - A0:: pure arabinoxylan spot (blank)
   - β0:: pure beta-glucan spot (blank)
   - A:: arabinoxylan spot after incubation with the enzyme noted below the track (BAN, Validase HT 425L and Alcalase AF 2.4L)
   - β:: beta-glucan spot after incubation with the enzyme noted below the track (BAN, Validase HT 425L and Alcalase AF 2.4L)
   - E0:: enzyme spot (blank)
Figure 2 shows size exclusion chromatography (SEC) of β-Glucan and arabinoxylan molecular weight profile without enzyme addition (plain line) and after incubation with Alcalase 2.4L (dotted line). A) Oat β-glucan; B) Wheat arabinoxylan.
Figure 3 shows size exclusion chromatography (SEC) of β-Glucan and arabinoxylan molecular weight profile without enzyme addition (plain line) and after incubation with Validase HT 425L (dotted line). A) Oat β-glucan; B) Wheat arabinoxylan.
Figure 4 shows size exclusion chromatography (SEC) of β-Glucan and arabinoxylan molecular weight profiles without enzyme addition (plain line) and after incubation with MATS L (dotted line). A) Oat β-glucan; B) Wheat arabinoxylan.
Figure 5 shows a comparison of soups with or without a hydrolyzed whole grain composition according to the invention. A) Soup without whole grains; B) Soup comprising 1 serving of whole grain with hydrolyzed whole wheat flour; and C) Soup comprising 1 serving of whole grain with un-processed whole wheat flour.

### DETAILED DESCRIPTION OF THE INVENTION

The inventors of the present invention have surprisingly found that by treating the whole grain component with an alpha-amylase and optionally with a protease the viscosity of the whole grain may be decreased and the following mixing into the food ingredient may be easier. This results in the possibility to increase the amount of whole grains in the product. Furthermore, the alpha-amylase treatment also results in a reduced need for adding sweetener such as sucrose to the food ingredient.

Thus in a first aspect the invention relates to a food ingredient comprising:
- a savoury flavour component;
- a hydrolyzed whole grain composition; and
- an alpha-amylase or fragment thereof, which alpha-amylase or fragment thereof shows no hydrolytic activity towards dietary fibers when in the active state;
   wherein the hydrolyzed whole grain composition has a substantially intact beta-glucan structure relative to the starting material and/or a substantially intact arabinoxylan structure relative to the starting material.

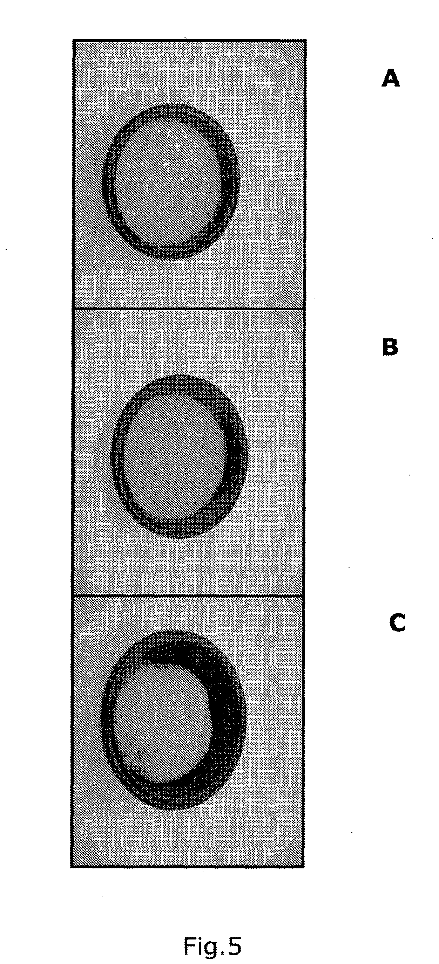

Several advantages of having a food ingredient comprising a hydrolyzed whole grain composition as well as composite food products comprising the food ingredient according to the invention may exist:
I. An increase in whole grain and fiber content may be provided in the final product, while the organoleptic parameters of the product are substantially not affected (see also figure 5);
II. Dietary fibers from the whole grain may be preserved;
III. Greater sense of satiety without significantly affecting the organoleptic parameters of the product and slower digestion. Currently, there are limitations for enriching food ingredients with whole grain due to non-flowable viscosity, grainy texture, and taste issues. However, the use of hydrolyzed whole grain according to the present invention in food ingredients allows for providing the desired viscosity, a smooth texture, minimal flavor impact, and added nutritional health and wellness values;
IV. An additional advantage may be to improve the carbohydrate profile of the food ingredient by replacing traditional externally supplied sweeteners such as glucose syrup, high fructose corn syrup, invert syrup, maltodextrine, sucrose, fiber concentrate, inulin, etc. with a more wholesome sweetener source.

In the present context the term "food ingredient" relates to a substance or a part of a composite food product. In an embodiment of the present invention the food ingredients may be a sauce, a soup, a pizza topping, a sandwich topping, a sandwich filling, or a dough filling.

In the present context the term "savoury flavour component" relates to any substance that imparts to or alters the savoury or umami taste and/or smell characteristics of the food ingredient and includes, but is not limited to, a seasoning agent, a spice, a herb, a fruit or a vegetable, or any mix of one or more seasoning agents, spices, herbs, fruits or vegetables.

In the present context the term "spice" relates to a dried seed, fruit, root, bark, or vegetative substance used in nutritionally insignificant quantities as a food additive for flavor and optionally for color. In the kitchen, spices are distinguished from herbs, which are leafy, green plant parts used for flavoring. Thus, in an embodiment the spice is, or is obtained from, dried seed, dried fruit, dried root, dried bark, dried vegetative substance or any combination thereof.

In a further embodiment the spice is pepper. In yet another embodiment the pepper is selected from the group consisting of the Piperaceae family, such as black pepper, white pepper, red pepper, and green pepper; the Solanaceae family, such as chili pepper, and cayenne pepper or combinations thereof.

The food ingredient may be in different forms. Thus, in another embodiment the food ingredient is in the form of a powder, a concentrate, a purée, a gel or a liquid, such as a frozen liquid. In yet an embodiment the food ingredient may be stored at ambient, chilled (such as 2-8°C) or under frozen conditions.

A quality parameter of the food ingredient and an important parameter in respect of the product processability is the viscosity of the hydrolysed whole grain composition. In the present context the term "viscosity" is a measurement of "thickness" or fluidability of a fluid. Thus, viscosity is a measure of the resistance of a fluid which is being deformed by either shear stress or tensile stress. If not indicated otherwise viscosity is given in mPa.s.

Viscosity may be measured using a Rapid Visco Analyser from Newport Scientific. The Rapid Visco Analyser measures the resistance of the product to the stirring action of a paddle. The viscosity is measured at 10 minutes stirring, at 65°C and 50 rpm.

The whole grain component may be obtained from different sources. Examples of whole grain sources are semolina, cones, grits, flour and micronized grain (micronized flour). The whole grains may be ground, preferably by dry milling. Such grinding may take place before or after the whole grain component is in contact with the enzyme composition according to the invention.

In an embodiment of the present invention the whole grain component may be heat treated to limit rancidity and microbial count.

Whole grains are cereals of monocotyledonous plants of the Poaceae family (grass family) cultivated for their edible, starchy grains. Examples of whole grain cereals include barley, rice, black rice, brown rice, wild rice, buckwheat, bulgur, corn, millet, oat, sorghum, spelt, triticale, rye, wheat, wheat berries, teff, canary grass, Job's tears and fonio. Plant species that do not belong to the grass family also produce starchy seeds or fruits that may be used in the same way as cereal grains, are called pseudo-cereals. Examples of pseudo-cereals include amaranth, buckwheat, tartar buckwheat and quinoa. When designating cereals, this will include both cereal and pseudo-cereals.

Thus, the whole grain component according to the invention may originate from a cereal or a pseudo-cereal. Thus, in an embodiment the hydrolyzed whole grain composition is obtained from a plant selected from the group consisting of barley, rice, brown rice, wild rice, black rice, buckwheat, bulgur, corn, millet, oat, sorghum, spelt, triticale, rye, wheat, wheat berries, teff, canary grass, Job's tears, fonio, amaranth, buckwheat, tartar buckwheat, quinoa, other variety of cereals and pseudo-cereals and mixtures thereof. In general the source of grain depends on the product type, since each grain will provide its own taste profile.

Whole grain components are components made from unrefined cereal grains. Whole grain components comprise the entire edible parts of a grain; i.e. the germ, the endosperm and the bran. Whole grain components may be provided in a variety of forms such as ground, flaked, cracked or other forms, as is commonly known in the milling industry.

In the present context the phrasing "a hydrolyzed whole grain composition" refers to enzymatically digested whole grain components or a whole grain component digested by using at least an alpha-amylase, which alpha-amylase shows no hydrolytic activity towards dietary fibers when in the active state. The hydrolyzed whole grain composition may be further digested by the use of a protease, which protease shows no hydrolytic activity towards dietary fibers when in the active state.

In the present context it is also to be understood that the phrase "a hydrolyzed whole grain composition" is also relating to enzymatic treatment of flour and subsequent reconstitution of the whole grain by blending flour, bran and germ. It is also to be understood that reconstitution may be done before the use in the final product or during mixing in a final product. Thus, reconstitution of whole grains after treatment of one or more of the individual parts of the whole grain also forms part of the present invention.

Prior to or after grinding of the whole grain, the whole grain component may be subjected to a hydrolytic treatment in order to breakdown the polysaccharide structure and optionally the protein structure of the whole grain component.

The hydrolyzed whole grain composition may be provided in the form of a liquid, a concentrate, a powder, a juice or a puree. If more than one type of enzymes is used it is to be understood that the enzymatic processing of the whole grains may be performed by sequential addition of the enzymes, or by providing an enzyme composition comprising more than one type of enzyme.

In the present context the phrase "an enzyme showing no hydrolytic activity towards dietary fibers when in the active state" should be understood as also encompassing the enzyme mixture from which the enzyme originates. For example, the proteases, amylases, glucose isomerase and amyloglucosidase described in the present context may be provided as an enzyme mixture before use which is not completely purified and thus, comprise enzymatic activity towards e.g. dietary fibers. However, the activity towards dietary fibers may also come from the specific enzyme if the enzyme is multi-functional. As used in here, the enzymes (or enzyme mixtures) are devoid of hydrolytic activity towards dietary fibers.

The term "no hydrolytic activity" or "devoid of hydrolytic activity towards dietary fibers" may encompass up to 5% degradation of the dietary fibers, such as up to 3%, such as up to 2% and such as up to 1% degradation. Such degradation may be unavoidable if high concentrations or extensive incubation times are used.

The term "in the active state" refers to the capability of the enzyme or enzyme mixture to perform hydrolytic activity, and is the state of the enzyme before it is deactivated. Deactivation may occur both by degradation and denaturation.

In general the weight percentages throughout the application are given as percentage by weight on a dry matter basis unless otherwise stated.

The food ingredient according to the invention may comprise a protease which shows no hydrolytic activity towards dietary fibers when in the active state. The advantage of adding a protease according to the invention is that the viscosity of the hydrolyzed whole grain may be further lowered, which may also result in a decrease in the viscosity of the final product. Thus, in an embodiment according to the invention the food ingredient comprises said protease or fragment thereof at a concentration of 0.0001 to 5% by weight of the total whole grain content, such as 0.01-3%, such as 0.01-1%, such as 0.05-1%, such as 0.1-1%, such as 0.1-0.7%, or such as 0.1-0.5%. The optimal concentration of added proteases depends on several factors. As it has been found that the addition of proteases during production of the hydrolyzed whole grain may result in a bitter off-taste, addition of proteases may be considered as a tradeoff between lower viscosity and off-taste. In addition the amount of protease may also depend on the incubation time during production of the hydrolyzed whole grain. For example, a lower concentration of protease may be used if the incubation time is increased.

Proteases are enzymes allowing the hydrolysis of proteins. They may be used to decrease the viscosity of the hydrolyzed whole grain composition. Alcalase 2.4L (EC 3.4.21.62), from Novozymes is an example of a suitable enzyme.

Depending on the incubation time and concentration of protease a certain amount of the proteins from the hydrolyzed whole grain component may be hydrolyzed to amino acid and peptide fragments. Thus, in an embodiment 1-10% of the proteins from the whole grain composition is hydrolyzed, such as 2-8%, e.g. 3-6%, 10-99%, such as 30-99%, such as 40-99%, such as 50-99%, such as 60-99%, such as 70-99%, such as 80-99%, such as 90-99%, or such as 10-40%, 40-70%, and 60-99%. Again protein degradation may result in a lowered viscosity and improved organoleptic parameters.

In the present context the phrase "hydrolyzed protein content" refers to the content of hydrolyzed protein from the whole grain composition unless otherwise defined. The protein may be degraded into larger or smaller peptide units or even into amino acid components. The person skilled in the art will know that during processing and storage small amount of degradation will take place which is not due to external enzymatic degradation.

In general it is to be understood that the enzymes used in the production of the hydrolyzed whole grain composition (and therefore also present in the final product) are different from the corresponding enzymes naturally present in the whole grain component.

Since the food ingredient according to the invention may also comprise proteins from sources, different from the hydrolyzed whole grain component, which are not degraded, it may be appropriate to evaluate the protein degradation on more specific proteins present in the whole grain composition. Thus, in an embodiment the degraded proteins are whole grain proteins, such as gluten proteins, globulins, albumins and glycoproteins.

Amylase (EC 3. 2. 1. 1) is an enzyme classified as a saccharidase: an enzyme that cleaves polysaccharides. It is mainly a constituent of pancreatic juice and saliva, needed for the breakdown of long-chain carbohydrates such as starch, into smaller units. Here, alpha-amylase is used to hydrolyse gelatinized starch in order to decrease the viscosity of the hydrolyzed whole grain composition. Validase HT 425L, Validase RA from Valley Research, Fungamyl from Novozymes and MATS from DSM are examples of alpha-amylases suitable for the present invention. Those enzymes show no activity towards the dietary fibers in the processing conditions used (duration, enzyme concentrations). On the contrary, e.g. BAN from Novozymes degrades dietary fibers besides starch into low molecular weight fibers or oligosaccharides, see also example 3.

In an embodiment of the present invention the enzymes show no activity towards the dietary fibers when the enzyme concentration is below 5% (w/w), such as below, 3% (w/w), e.g. below 1% (w/w), such as below 0.75% (w/w), e.g. below 0.5% (w/w).

Some alpha-amylases generate maltose units as the smallest carbohydrate entities, whereas others are also able to produce a fraction of glucose units. Thus, in an embodiment the alpha-amylase or fragment thereof is a mixed sugar producing alpha-amylase, including glucose producing activity, when in the active state. It has been found that some alpha-amylases both comprise glucose producing activity while having no hydrolytic activity towards dietary fibers when in the active state. By having an alpha-amylase which comprises glucose producing activity an increased sweetness may be obtained, since glucose has almost twice the sweetness of maltose. In an embodiment of the present invention a reduced amount of external sugar source may be added to the food ingredient when a hydrolysed whole grain composition according to the present invention is used. When an alpha-amylase comprising glucose producing activity is used in the enzyme composition, it may become possible to dispense or at least reduce the use of other external sugar sources or non-sugar sweeteners.

In the present context the term "external sugar source" relates to sugars not originally present or originally generated in the hydrolysed whole grain composition. Examples of such external sugar source could be sucrose, lactose, and artificial sweeteners.

Amyloglucosidase (EC 3.2.1.3) is an enzyme able to release glucose residues from starch, maltodextrins and maltose by hydrolysing glucose units from the non-reduced end of the polysaccharide chain. The sweetness of the preparation increases with increasing concentration of released glucose. Thus, in an embodiment the food ingredient further comprises an amyloglucosidase or fragment thereof. It may be advantageous to add an amyloglucosidase to the production of the hydrolyzed whole grain composition, since the sweetness of the preparation increases with the increasing concentration of released glucose. It may also be advantageous if the amyloglucosidase does not influence health properties of the whole grains, directly or indirectly. Thus, in an embodiment the amyloglucosidase shows no hydrolytic activity towards dietary fibers when in the active state. An interest of the invention, and particularly of the process for preparing the food ingredient according to the invention, is that it allows reducing the sugar (e.g. sucrose) content of the food ingredient when compared to other known products. When an amyloglucosidase is used in the enzyme composition, it may become possible to dispense with other external sugar sources e.g. the addition of sucrose.

However, as mentioned above certain alpha-amylases are able to generate glucose units, which may add enough sweetness to the product making the use of amyloglucosidase dispensable. Furthermore, application of amyloglucosidase also increases production costs of the food ingredient and, hence, it may be desirable to limit the use of amyloglucosidases. Thus, in yet an embodiment the food ingredient according to the invention does not comprise an amyloglucosidase such as an exogenic amyloglucosidase.

Glucose isomerase (D-glucose ketoisomerase) causes the isomerization of glucose to fructose. Thus, in an embodiment of the present invention the food ingredient further comprises a glucose isomerase or fragment thereof, which glucose isomerase or fragment thereof shows no hydrolytic activity towards dietary fibers when in the active state. Glucose has 70-75% the sweetness of sucrose, whereas fructose is twice as sweet as sucrose. Thus, processes for the manufacture of fructose are of considerable value because the sweetness of the product may be significantly increased without the addition of an external sugar source (such as sucrose or artificial sweetening agents).

A number of specific enzymes or enzyme mixtures may be used for production of the hydrolyzed whole grain composition according to the invention. The requirement is that they show substantially no hydrolytic activity in the process conditions used towards dietary fibers. Thus, in an embodiment the alpha-amylase may be selected from Validase HT 425L and Validase RA from Valley Research, Fungamyl from Novozymes and MATS from DSM, the protease may be selected from the group consisting of Alcalase, iZyme B and iZyme G (Novozymes).

According to the invention the concentration of the enzymes in the food ingredient may influence the organoleptic parameters of the food ingredient. In addition the concentration of enzymes may also be adjusted by changing parameters such as temperature and incubation time. Thus, in an embodiment the food ingredient comprises 0.0001 to 5% by weight of the total whole grain content in the food ingredient of at least one of:
- an alpha-amylase or fragment thereof, which alpha-amylase or fragment thereof shows no hydrolytic activity towards dietary fibers when in the active state;
- an amyloglucosidase or fragment thereof, which amyloglucosidase shows no hydrolytic activity towards dietary fibers when in the active state; and
- a glucose isomerase or fragment thereof, which amyloglucosidase shows no hydrolytic activity towards dietary fibers when in the active state.

In yet another embodiment the food ingredient comprises 0.001 to 3% of the alpha-amylase by weight of the total whole grain content in the food ingredient, such as 0.01-3%, such as 0.01-0.1%, such as 0.01-0.5%, such as 0.01-0.1%, such as 0.03-0.1%, such as 0.04-0.1%. In yet another embodiment the food ingredient comprises 0.001 to 3% of the amyloglucosidase by weight of the total whole grain content in the food ingredient, such as 0.001-3%, such as 0.01-1%, such as 0.01-0.5%, such as 0.01-0.5%, such as 0.01-0.1%, such as 0.03-0.1%, such as 0.04-0.1%. In another further embodiment the food ingredient comprises 0.001 to 3% of the glucose isomerase by weight of the total whole grain content in the food ingredient, such as 0.001-3%, such as 0.01-1%, such as 0.01-0.5%, such as 0.01-0.5%, such as 0.01-0.1%, such as 0.03-0.1%, such as 0.04-0.1%.

Beta-amylases are enzymes which also break down saccharides, however beta-amylases mainly have maltose as the smallest generated carbohydrate entity. Thus, in an embodiment the food ingredient according to the invention does not comprise a beta-amylase, such as an exogenic beta-amylase. By avoiding beta-amylases a larger fraction of the starches will be hydrolyzed to glucose units since the alpha-amylases do have to compete with the beta-amylases for substrates. Thus, an improved sugar profile may be obtained. This is in contrast to US 5,686,123 which discloses a cereal suspension generated by treatment with both an alpha-amylase and a beta-amylase.

In certain instances the action of the protease is not necessary, to provide a sufficiently low viscosity. Thus, in an embodiment according to the invention, the food ingredient does not comprise the protease, such as an exogenic protease. As described earlier the addition of protease may generate a bitter off-taste which in certain instances is desirable to avoid. This is in contrast to US 4,282,319 which discloses a process including enzymatic treatment with a protease and an amylase.

In general the enzymes used according to the present invention for producing the hydrolyzed whole grain composition show no hydrolytic activity towards dietary fibers when in the active state. Thus, in a further embodiment the hydrolyzed whole grain composition has a substantially intact beta-glucan structure relative to the starting material. In yet a further embodiment the hydrolyzed whole composition has a substantially intact arabinoxylan structure relative to the starting material. By using the one or more enzymes according to the invention for the production of the hydrolyzed whole grain composition, a substantially intact beta-glucan and arabinoxylan structure may be maintained. The degree of degradation of the beta-glucan and arabinoxylan structures may be determined by Size-exclusion chromatography (SEC). This SEC technique has been described in more detail in "Determination of beta-Glucan Molecular Weight Using SECwith Calcofluor Detection in Cereal Extracts Lena Rimsten, Tove Stenberg, Roger Andersson, Annica Andersson, and Per Åman. Cereal Chem. 80(4):485-490",which is hereby incorporated by reference.

In the present context the phrase "substantially intact structure" is to be understood as for the most part the structure is intact. However, due to natural degradation in any natural product, part of a structure (such as beta-glucan structure or arabinoxylan structure) may be degraded although the degradation may not be due to added enzymes. Thus, "substantially intact structure" is to be understood that the structure is at least 95% intact, such as at least 97%, such as at least 98%, or such as at least 99% intact.

In the present context enzymes such as proteases, amylases, glucose isomerases and amyloglucosidases refer to enzymes which have been previously purified or partly purified. Such proteins/enzymes may be produced in bacteria, fungi or yeast, however they may also have plant origin. In general such produced enzymes will in the present context fall under the category "exogenic enzymes". Such enzymes may be added to a product during production to add a certain enzymatic effect to a substance. Similar, in the present context, when an enzyme is disclaimed from the present invention such disclaimer refers to exogenic enzymes. In the present context such enzymes e.g. provide enzymatic degradation of starch and proteins to decrease viscosity. In relation to the process of the invention it is to be understood that such enzymes may both be in solution or attached to a surface, such as immobilized enzymes. In the latter method the proteins may not form part of the final product.

As mentioned earlier, the action of the alpha-amylase results in a useful sugar profile which may affect taste and reduce the amount of external sugar or sweetener to be added to the final product.

In an embodiment of the present invention the hydrolysed whole grain composition has a glucose content of at least 0.25% by weight of the hydrolysed whole grain composition, on a dry matter.basis, such as at least 0.35%, e.g. at least 0.5%.

Depending on the specific enzymes used the sugar profile of the final product may change. Thus, in an embodiment the food ingredient has a maltose to glucose ratio below 144:1, by weight in the product, such as below 120:1, such as below 100:1 e.g. below 50:1, such as below 30:1, such as below 20:1 or such as below 10:1.

If the only starch processing enzyme used is a glucose generating alpha-amylase, a larger fraction of the end product will be in the form of glucose compared to the use of an alpha-amylase specifically generating maltose units. Since glucose has a higher sweetness than maltose, this may mean that the addition of a further sugar source (e.g. sucrose) can be dispensed. This advantage may be further pronounced if the ratio is lowered by the conversion of the maltose present in the hydrolyzed whole grain to glucose (one maltose unit is converted to two glucose units).

The maltose to glucose ratio may be further lowered if an amyloglucosidase is included in the enzyme composition since such enzymes also generates glucose units.

If the enzyme composition comprises a glucose isomerase a fraction of the glucose is changed to fructose which has an even higher sweetness than glucose. Thus, in an embodiment the food ingredient has a maltose to glucose + fructose ratio below 144:1 by weight in the product, such as below 120:1, such as below 100:1 e.g. below 50:1, such as below 30:1, such as below 20:1 or such as below 10:1.

Furthermore, in an embodiment of the present invention the food ingredient may have a maltose to fructose ratio below 230:1 by weight in the product, such as below 144:1, such as below 120:1, such as below 100:1 e.g. below 50:1, such as below 30:1, such as below 20:1 or such as below 10:1.

In the present context the phrasing "total content of the whole grain" is to be understood as the combination of the content of "hydrolyzed whole grain composition" and "solid whole grain content". If not indicated otherwise, "total content of the whole grain" is provided as % by weight in the final product. In an embodiment the food ingredient has a total content of the whole grain in the range of 1-35% by weight of the food ingredient such as in the range 10-30%; such as in the range 20-28%. such as 1-20%, such as 1-15%, such as 1-10%, and such as 1-7%.

In the present context the phrasing "content of the hydrolyzed whole grain composition" is to be understood as the % by weight of hydrolyzed whole grains in the final product. Hydrolyzed whole grain composition content is part of the total content of the whole grain composition. Thus, in an embodiment the food ingredient according to the invention has a content of the hydrolyzed whole grain composition in the range 1-35% by weight of the food ingredient such as in the range 3-18%, such as 5.5-16% such as 10-30%, such as in the range 20-28%. The amount of the hydrolyzed whole grain composition in the final product may depend on the type of product. By using the hydrolyzed whole grain composition according to the invention in a food ingredient, a higher amount of hydrolyzed whole grains may be added (compared to a non-hydrolyzed whole grain composition) without substantially affecting the organoleptic parameters of the product because of the increased amount of soluble fibers in the hydrolysed whole grain. A target amount of the hydrolyzed whole grain composition in the food ingredient may be 4-16 grams per serving. Serving of a sauce or soup typically is 40-300 grams.

It would be advantageous to have a food ingredient comprising a high content of dietary fibers without compromising the organoleptic parameters of the product. Thus, in yet another embodiment the food ingredient has a content of dietary fibers in the range of 0.1-10% by weight of the food ingredient, preferably, in the range of 0.5-3%, even more preferably in the range of 1-2% (w/w). A food ingredient according to the invention may be provided with high amounts of dietary fibers by the addition of the hydrolyzed whole grain component provided by the present invention. This may be done due to the unique setup of the process according to the present invention.

Dietary fibers are the edible parts of plants that are not broken down by digestion enzymes. Dietary fibers are fermented in the human large intestine by the microflora. There are two types of fibers: soluble fibers and insoluble fibers. Both soluble and insoluble dietary fibers can promote a number of positive physiological effects, including a good transit through the intestinal tract which helps to prevent constipation, or a feeling of fullness. Health authorities recommend a consumption of between 20 and 35 g per day of fibers, depending on the weight, gender, age and energy intake.

Soluble fibers are dietary fibers that undergo complete or partial fermentation in the large intestine. Examples of soluble fibers from cereals include beta-glucans, arabinoxylans, arabinogalactans and resistant starch type 2 and 3, and oligosaccharides deriving from the latters. Soluble fibers from other sources include pectins, acacia gum, gums, alginate, agar, polydextrose, inulins and galacto-oligosaccharides for instance. Some soluble fibers are called prebiotics, because they are a source of energy for the beneficial bacteria (e.g. Bifidobacteria and Lactobacilli) present in the large intestine. Further benefits of soluble fibers include blood sugar control, which is important in diabetes prevention, control of cholesterol, or risk reduction of cardiovascular disease.

Insoluble fibers are the dietary fibers that are not fermented in the large intestine or only slowly digested by the intestinal microflora. Examples of insoluble fibers include celluloses, hemicelluloses, resistant starch type 1 and lignins. Further benefits of insoluble fibers include promotion of the bowel function through stimulation of the peristalsis, which causes the muscles of the colon to work more, become stronger and function better. There is also evidence that consumption of insoluble fibers may be linked to a reduced risk of gut cancer.

The total moisture content of the food ingredient according to the invention may vary. Thus, in another embodiment the total moisture content is in the range of 20-95%, such as 35-85% and such as 40-85% by weight of the food ingredient. Examples of factors influencing the solid content may be the amount of the hydrolyzed whole grain composition and the degree of hydrolysis in this composition. In the case the food ingredient the moisture content may of course be lower. Thus, in yet an embodiment the food ingredient is a powder having a moisture content below 5%. In the present context the phrasing "total solid content" equals 100 minus moisture content (%) of the product.

It would be advantageous if a food ingredient with good organoleptic parameters, such as sweetness, could be obtained, without addition of large amounts of sugar from external sources compared to food ingredients without the hydrolyzed whole grain composition as described in the present invention. Thus, in another embodiment the food ingredient has a content of sweetening agent in the range of 0-50, such as in the range of 0.05-25%, e.g. in the range of 0.5-10%, such as in the range of 1-5% by weight of the food ingredient.

Since the hydrolyzed whole grain composition supplements the food ingredient and the subsequent composite food product with a source of carbohydrates, such as glucose and maltose, the food ingredient is also sweetened from a natural sugar source different from the external sugar source. Thus, the amount of added external sweetener may be limited. In an embodiment the sweetening agent is a sugar or a non-sugar sweetener. In another embodiment the sugar is a monosaccharide, a disaccharide, a sugar alcohol, an oligosaccharide or a combination hereof. In yet another embodiment the monosaccharide is glucose, galactose, fructose or any combination hereof. In a further embodiment the disaccharide is maltose, sucrose, lactose or any combination hereof. In a more specific embodiment the sugar is sucrose.

Sucrose is a widely used sweetener in food products, however other sugars may also be used such as brown sugar, molasses, brown sugar syrup and honey.

Humectants are often added to products which are to be in a dry or semi-dry state. Thus, in an embodiment the food ingredient does not comprise a humectant. Supplementary ingredients of the food ingredient include vitamins and minerals, preservatives such as tocopherol, and emulsifiers, such as lecithin, protein powders, cocoa solid, alkylresorcinols, phenolics and other active ingredients, such as DHA, caffeine, and prebiotics.

In a further embodiment the food ingredient has a fat content in the range 0.1-50% by weight of the food ingredient, such as in the range of, 0.1-30% by weight of the food ingredient, such as in the range of, 0.1-20% by weight of the food ingredient, such as 0.1-12%, such as 0.1-8%, such as 5-20%, and such as 10-17%. The amount of fat may vary depending on the type of product. Fat components are preferably vegetable fats such as cocoa butter, rapeseed oil, sunflower oil, soybean oil, olive oil, palm oil, diary fat, butter, cheese, milk, animal fat (such as chicken or beef fat), preferably not hydrogenated.

In yet another embodiment the food ingredient may have salt content in the range 0-8%, such as in the range of 1-6%, e.g. in the range of 2-4%, such as in the range of 2.1-3% by weight of the food ingredient. In a more specific embodiment the salt is sodium chloride.

Depending on the specific type of food ingredient, different flavor components may be added to provide the desired taste. Thus, in a further embodiment the flavor component is selected from the group consisting of dairy components (such as cheese, cheese flavors or paste, whey powder, milk fat), sesame oil, olive oil, canola oil, soy oil, butter, margarine, vegetable and fruit pieces, purees, paste, powder, rinds, juices or dried, bell pepper, onion, tomatoes, orange, lemon, lime, coconut, carrots, spinach, plum sauce, pineapple, smoked tomatoes, grilled zucchini, corn, roasted corn, fire roasted tomatoes, garlic, ginger, cilantro, fennel, tamarind paste, chile sauce, parsley, sesame seeds, soy sauce, teriyaki sauce, Hoisin sauce, mustard (flour/paste/ground), mushrooms, almonds, peanuts, walnuts, olives, Worcestershire sauce, garlic-chili sauce, curry, egg yolks powder, vanilla, vinegar (balsamic, cidar, or white), wine (Chablis, Sherry, Burgandy, Port, Madeira), lactic acid, citric acid, flavor enhancer, chicken flavor, shrimp flavor, coconut flavor, beef flavor, fried flavor, chicken fat flavor, garlic flavor, ginger flavor, grill flavor, mesquite flavor, cumin, mirepoix base or flavor, mushroom flavor, smoke flavor, bacon flavor, hickory flavor, cream flavor, beef au jus concentrate, chicken base, beef base, chicken concentrate, beef concentrate, beef broth, chicken broth, bacon, sea salt, salt, tamari sauce, yeast extract, potassium chloride and combinations thereof.

To improve the appearance of the product, the food ingredient according to the invention may also comprise a color component. Thus, in an embodiment the food ingredient comprises a color component. The flavor or spice component may also function as a color component. However, separate color component(s) may be added.

It may also be advantageous to improve the texture of the food ingredient. Thus, in an embodiment the food ingredient comprises a texturing agent. In yet another embodiment the texturing agent is added in an amount in the range of 0.01-10%, such as 0.05% to 6% by weight of the food ingredient. In a further embodiment the texturing agent is selected from the group consisting of corn, rice or wheat starch, modified corn or wheat starch, or wheat flour, and/or a hydrocolloid, such as xanthan gum, carageenan gum, pectin, or gelatin. The amount of the specific type of texturing agent may vary. Thus, in an embodiment the food ingredient comprises a modified starch in the range of 1.0-3.5 % such as 1-2.5% by weight of the food ingredient; wheat pastry flour in the range of 1.0-3% by weight of the food ingredient; xanthan gum in the range 0.05-0.5% by weight of the food ingredient or combinations thereof.

Depending on the specific type of product, the food ingredient may comprise or be reconstituted in a liquid component before consumption. The liquid may provide the right consistency, viscosity, taste, and nutritional profile. In a further embodiment the liquid component is water, milk, milk components, tomato puree, tomato paste, crushed, diced or whole tomatoes, tomato juice or mixtures hereof.

The present invention also provides a process for preparing a food ingredient, said process comprising:
1) preparing a hydrolyzed whole grain composition, comprising the steps of:
   a) contacting a whole grain component with an enzyme composition in water, said enzyme composition showing no hydrolytic activity towards dietary fibers,
   b) allowing the enzyme composition to react with the whole grain component, to provide a whole grain hydrolysate,
   c) providing the hydrolyzed whole grain composition by deactivating said enzymes when said hydrolysate has reached a viscosity comprised between 50 and 12000 mPa.s measured at 65°C,
2) providing the food ingredient by mixing the hydrolyzed whole grain composition with a premix comprising a savoury flavour component.

In an embodiment the enzyme composition further comprises a protease or fragment thereof, which protease or fragment thereof shows no hydrolytic activity towards dietary fibers when in the active state. Similar, the enzyme composition may comprise an amyloglucosidase and/or and glucose isomerase according to the present invention.

Several parameters of the process may be controlled to provide the food ingredient according to the invention. Thus, in an embodiment step 1b) is performed at 30-100°C, preferably 50 to 85°C. In a further embodiment step 1b) is performed for 1 minute to 24 hours, such as 1 minute to 12 hours, such as 1 minute to 6 hours, such as 5-120 minutes. In yet an embodiment step 1b) is performed at 30-100°C for 5-120 minutes.

In yet a further embodiment step 1c) is allowed to proceed at 70-150°C for at least 1 second, such as 1-5 minutes, such as 5-120 minutes, such as 5-60 minutes. In an additional embodiment step 1c) is performed by heating to at least 90°C for 5-30 minutes.

In yet another embodiment the reaction in step 1c) is stopped when the hydrolysate has reached a viscosity comprised between 50 and 5000 mPa.s, such as between 50 and 3000 mPa.s, such as between 50 and 1000 mPa.s, such as between 50 and 500 mPa.s. In an additional embodiment viscosity is measured at TS 50.

In another embodiment the hydrolyzed whole grain composition in step 1) is provided when said hydrolysate has reached a total solid content of 25-60%. By controlling viscosity and solid content the hydrolyzed whole grain may be provided in different forms.

In an additional embodiment the hydrolyzed whole grain component in step 1c) is provided in the form of a liquid, a concentrate, a powder, a juice or a puree. An advantage of having hydrolyzed whole grain composition in different forms is that when used in a food product dilution may be avoided by using a dry or semi dry form. Similarly, if a more moisten product is desirable, a hydrolyzed whole grain composition in a liquid state may be used.

The above parameters can be adjusted to regulate the degree of starch degradation, the sugar profile, the total solid content and to regulate the overall organoleptic parameters of the final product.

To improve the enzymatic processing of the whole grain component it may be advantageous to process the grains before or after the enzymatic treatment.

By grinding the grains a larger surface area is made accessible to the enzymes, thereby speeding up the process. In addition the organoleptic parameters may be improved by using a smaller particle size of the grains. In an additional embodiment the whole grains are roasted or toasted before or after enzymatic treatment. Roasting and toasting may improve the taste of the final product.

To prolong the storage time of the product several treatments can be performed. Thus, in an embodiment the process further comprises at least one of the following treatments: UHT, pasteurization, thermal treatment, retort and any other thermal or non-thermal treatments, such as pressure treatment. In a further embodiment the food ingredient is applied to an enclosure under aseptic conditions. In yet an embodiment the food ingredient is applied to an enclosure under non-aseptic conditions, such as by retort or hot-for-hold.

In another aspect the invention relates to composite food product comprising a food ingredient according to the invention. In an embodiment the composite food product may be a pasta with tomato sauce, butternut squash soup with a ham sandwich, meal of chicken with gravy, vegetables, and pasta, meal of chicken with sesame sauce and vegetables, meal of salmon with vegetables and basil cream sauce, a topping for pizza, a filled closed sandwich such as a pepperoni or cheese filled Hot Pocket.

In an embodiment of the present invention the composite food product may be stored at ambient temperatures, chilled temperatures, such as at 2-8°C, or under frozen conditions. Preferably, the composite food product may be stored under frozen conditions.

It should be noted that embodiments and features described in the context of one of the aspects or embodiments of the present invention also apply to the other aspects of the invention.

All patent and non-patent references cited in the present application, are hereby incorporated by reference in their entirety.

The invention will now be described in further detail in the following non-limiting examples.

### Examples

### Example 1 - Preparation of a hydrolysed whole grain composition

Enzyme compositions comprising Validase HT 425L (alpha-amylase) optionally in combination with Alcalase 2.4 L (protease) were used for the hydrolysis of wheat, barley and oats.

Mixing may be performed in a double jacket cooker, though other industrial equipment may be used. A scraping mixer works continuously and scraps the inner surface of the mixer. It avoids product burning and helps maintain a homogeneous temperature. Thus enzyme activity is better controlled. Steam may be injected in the double jacket to increase the temperature while cold water may be used to decrease the temperature.

In an embodiment, the enzyme composition and water are mixed together at a temperature, between 10 and 25°C. At this low temperature, the enzymes of the enzyme composition have a very weak activity. The whole grain component is then added and the ingredients are mixed for a short period of time, usually less than 20 minutes, until the mixture is homogeneous.

The mixture is heated progressively or by thresholds to activate the enzymes and hydrolyse the whole grain component.

Hydrolysis results in a reduction of the viscosity of the mixture. When the whole grain hydrolysate has reached a viscosity between 50 and 5000 mPa.s measured at 65°C and e.g. a total solid content of 25 to 60% by weight, the enzymes are deactivated by heating the hydrolysate at a temperature above 100°C, preferably by steam injection at 120°C.

Enzymes are dosed according to the quantity of total whole grain. Quantities of enzymes are different depending on the type of whole grain component, as protein rates are different. The ratio of water/whole grain component can be adapted according to required moisture for the final liquid whole grain. Usually, the water/whole grain component ratio is 60/40. Percentages are by weight.

| Hydrolysed whole wheat | |
|---|---|
| Whole wheat flour | Substrate |
| Enzyme amylase | 0.10% based on the substrate |
| Enzyme protease | 0.05% based on the substrate |

| Hydrolysed whole barley | |
|---|---|
| Whole barley flour | Substrate |
| Enzyme amylase | 0.10% based on the substrate |
| Enzyme protease | 0.05% based on the substrate |

| Hydrolysed whole oats | |
|---|---|
| Whole oat flour | Substrate |
| Enzyme amylase | 0.10% based on the substrate |
| Enzyme protease | 0.05% based on the substrate |

### Example 2 - Sugar profile of the hydrolyzed whole grain composition

Hydrolyzed whole grain compositions comprising wheat, barley and oats were prepared according to the method in example 1.

### Carbohydrates HPAE:

The hydrolyzed whole grain compositions were analysed by HPAE for illustrating the sugar profile of hydrolysed whole grain compositions.

Carbohydrates are extracted with water, and separated by ion chromatography on an anion exchange column. The eluted compounds are detected electrochemically by means of a pulsed amperometric detector and quantified by comparison with the peak areas of external standards.

### Total dietary fibres:

Duplicate samples (defatted if necessary) are digested for 16 hours in a manner that simulates the human digestive system with 3 enzymes (pancreatic alpha-amylase, protease, and amyloglucosidase) to remove starch and protein. Ethanol is added to precipitate high molecular weight soluble dietary fibre. The resulting mixture is filtered and the residue is dried and weighed. Protein is determined on the residue of one of the duplicates; ash on the other. The filtrate is captured, concentrated, and analyzed via HPLC to determine the value of low molecular weight soluble dietary fibre (LMWSF).

*Whole wheat:*

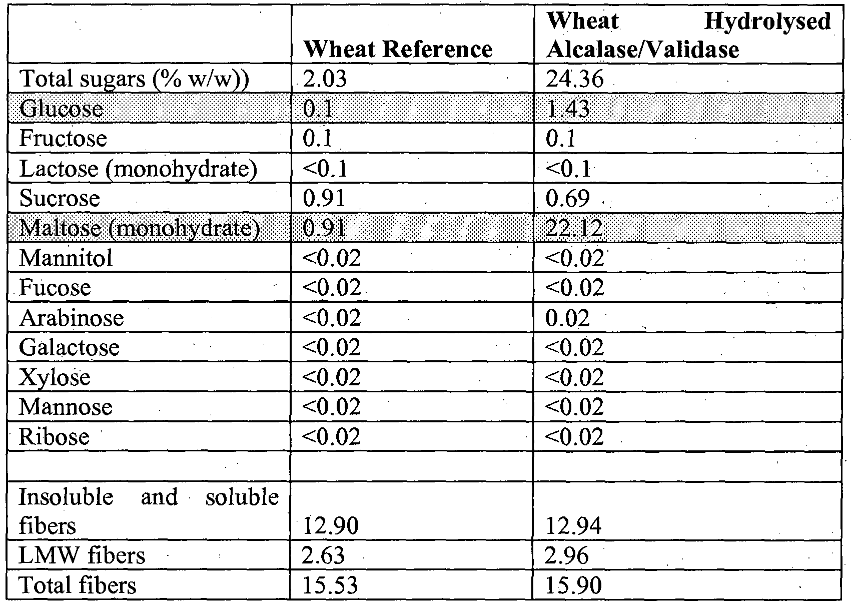

### Whole oats:

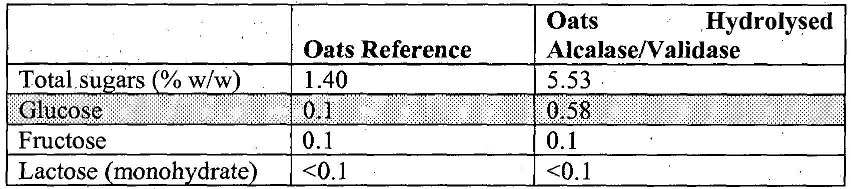

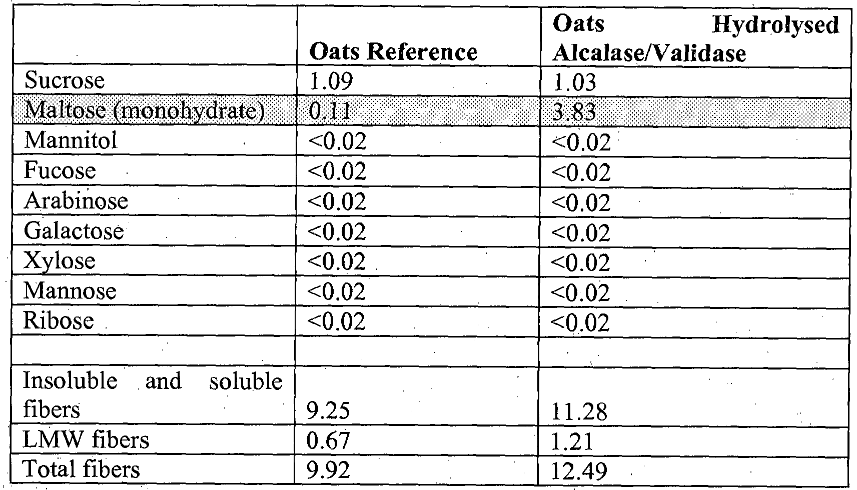

### Whole Barley:

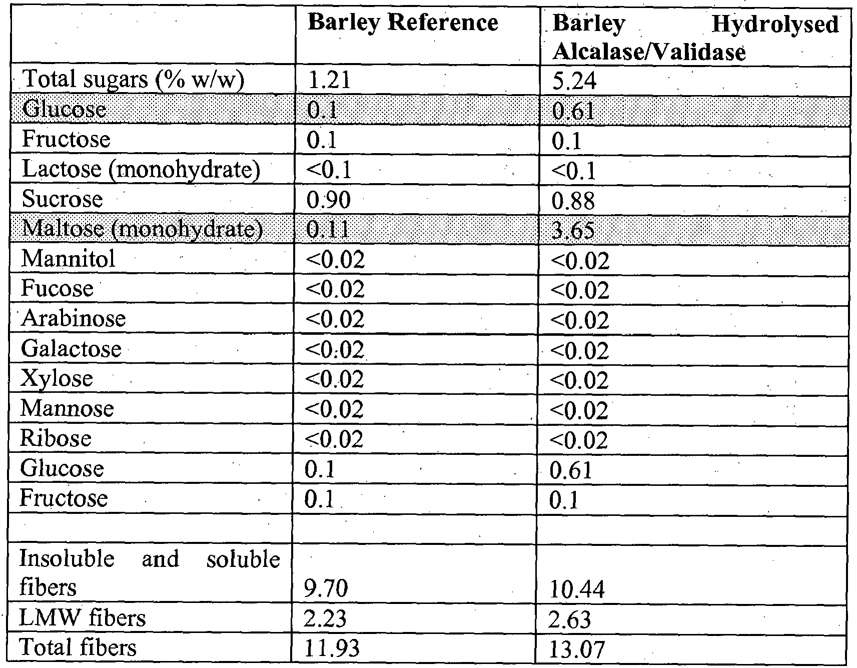

The results clearly demonstrate that a significant increase in the glucose content is provided by the hydrolysis where the glucose content of the hydrolysed barley is 0.61% (w/w) on a dry matter basis; the glucose content of the hydrolysed oat is 0.58% (w/w) on a dry matter basis; and the glucose content of the hydrolysed wheat is 1.43% (w/w) on a dry matter basis.

Furthermore, the results also demonstrates that the maltose:glucose ratio is in the range from about 15:1 to about 6:1.

Thus, based on these results a new sugar profile is provided having a increased sweetness compared to the prior art.

In conclusion, an increased sweetness may be obtained by using the hydrolyzed whole grain composition according to the invention and therefore the need for further sweetening sources may be dispensed or limited.

In addition, the results demonstrate that the dietary fiber content is kept intact and the ratio and amount of soluble and insoluble fibers are substantially the same in the non-hydrolyzed whole grain and in the hydrolyzed whole grain composition.

### Example 3 - Hydrolytic activity on dietary fibers

The enzymes Validase HT 425L (Valley Research), Alcalase 2.4L (Novozymes) and BAN (Novozymes) were analysed using a thin layer chromatography analysis for activity towards arabinoxylan and beta-glucan fibre extracts both components of dietary fibers of whole grain.

The results from the thin layer chromatography analysis showed that the amylase Validase HT and the protease Alcalase showed no hydrolytic activity on either beta-glucan or arabinoxylan, while the commercial alpha-amylase preparation, BAN, causes hydrolysis of both the beta-glucan and arabinoxylan, see figure 1.

See also example 4.

### Example 4 - Oat β-Glucan and Arabinoxylan molecular weight profile following enzymatic hydrolysis

### Hydrolysis:

A solution of 0.5 % (w/v) of Oat β-Glucan medium viscosity (Megazyme) or Wheat Arabinoxylan medium viscosity (Megazyme) was prepared in water.

The enzyme was added at an enzyme to substrate ratio (E/S) of 0.1 % (v/v). The reaction was allowed to proceed at 50°C for 20 minutes, the sample was then placed at 85°C during 15 min to enable starch gelatinization and hydrolysis. The enzymes were finally inactivated at 95°C for 15 minutes. Different batches of the following enzymes have been evaluated.

| | |
|---|---|
| Alcalase 2.4L (Valley Research): | batch BN 00013 |
| | batch 62477 |
| | batch 75039 |
| Validase HT 425L (Valley Research): | batch RA8303A |
| | batch 72044 |
| MATS L (DSM): | batch 408280001 |

### Molecular weight analysis

Hydrolyzed samples were filtered on a syringe filter (0.22 µm) and 25 µL were injected on a High Pressure Liquid Chromatography Agilent 1200 series equipped with 2 TSKgel columns in serie (G3000PWXL 7,8 x 300 mm), (GMPWXL 7,8X 30 mm) and with a guard column (PWXL 6 x 44 mm). (Tosoh Bioscence) Sodium Nitrate 0.1M/ at 0.5ml/min was used as running buffer. Detection was done by reflective index measurement.

### Results

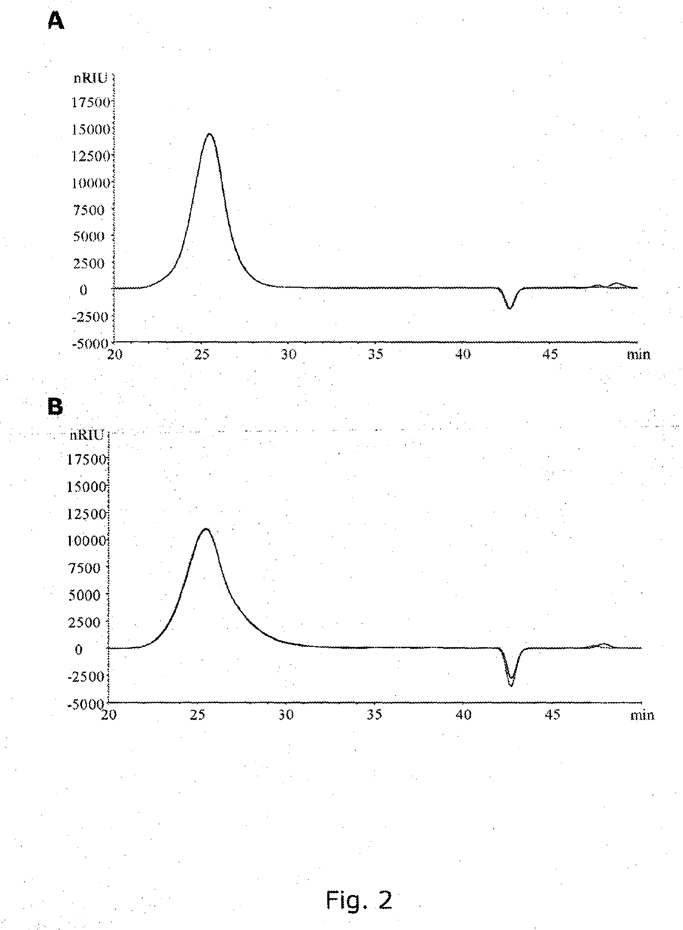

On figures 2-4 graphs for both a control (no enzyme) and test with enzymes are plotted. However, since there are substantially no difference between the graphs it may be difficult to differentiate both graphs from each other.

### Conclusions

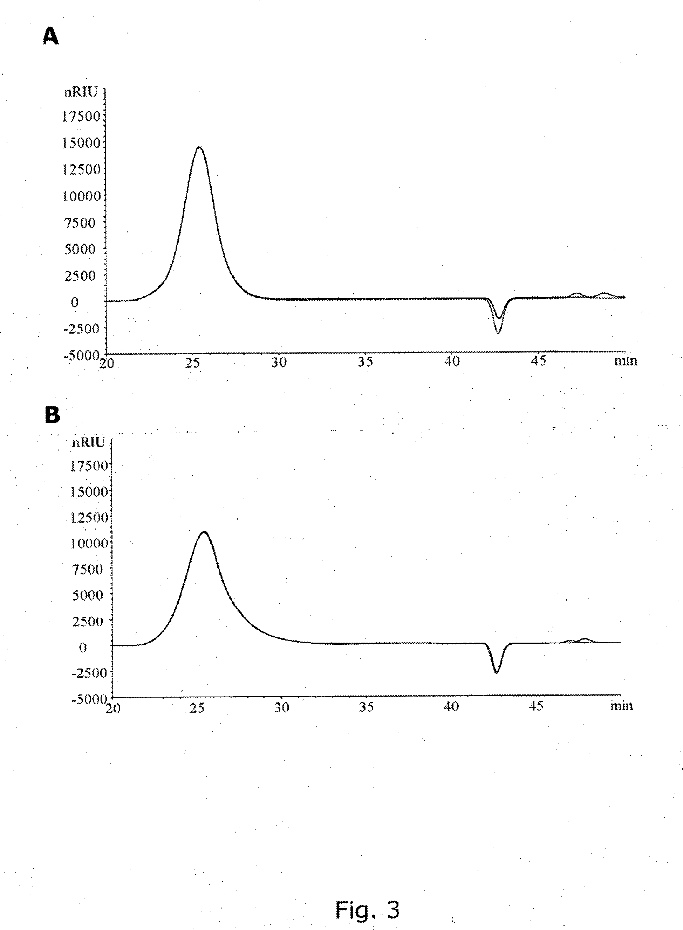

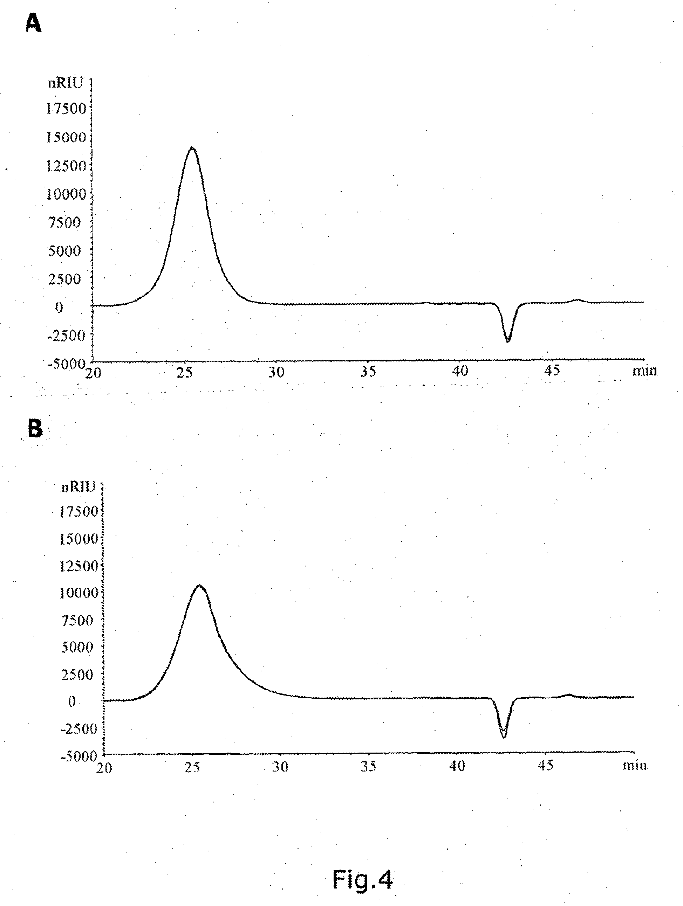

No shift in oat beta glucan and wheat arabinoxylan fibre molecular weight profile was determined following hydrolysis with the Alcalase 2.4 L (figure 2), Validase HT 425 L (figure 3) or MATS L (figure 4).

### Example 5 - Squash soup comprising a hydrolyzed whole grain composition

The hydrolyzed whole grain composition (HWGC) is prepared according to example 1.

| Squash Soup | | **Reference** | **HWGC added to Slurry** | **HWGC added to Mix Batch** |
|---|---|---|---|---|
| | **Ingredient** | | | |
| | | **%** | **%** | **%** |
| Slurry | | | | |
| | Milk | 35.80 | 26.34 | 35.80 |
| | Oil Olive | 2.60 | 2.000 | 2.60 |
| | Corn Starch | 0.50 | 0.430 | 0.50 |
| | Salt | 0.55 | 0.500 | 0.55 |
| | Spices (red pepper, black pepper, cinnamon, orange peel) | 0.05 | 0.030 | 0.05 |
| | Squash Puree | 54.50 | 44.000 | 54.50 |
| | Onion Puree | 6.00 | 5.200 | 6.00 |
| | HWGC Wheat (50% moisture) | 0.00 | 21.500 | 0.00 |
| SUBTOTAL | | 100.00 | 100.000 | 100.00 |
| | | | | |
| Mix Batch | | | | |
| | Slurry Batch | 0.00 | 0.000 | 78.50 |
| | HWGC Wheat (50 % moisture) | 0.00 | 0.000 | 21.50 |
| | | | | |
| FINAL PRODUCT | | | | |
| | Total input ingredients | 100.00 | 100.000 | 100.00 |
| | Per pkg. | 150gm | 150gm | 150gm |

In addition, Figure 5 shows a comparison of soups with or without a hydrolyzed whole grain composition according to the invention. A) Soup without whole grains; B) Soup comprising whole grain with hydrolyzed whole wheat flour; and C) Soup comprising of whole grain with un-processed whole wheat flour. Thus, by using the hydrolyzed whole grain composition according to the invention the organoleptic parameters of e.g. a soup may be improved.

### Example 6 - Tomato sauce width pasta

The present example relates both to a I) composite food product according to the invention comprising a food ingredient (tomato sauce) comprising a hydrolyzed whole grain composition and pasta and II) a food ingredient (tomato sauce) comprising a hydrolyzed whole grain composition.

The hydrolyzed whole grain composition (HWGC) is prepared according to example 1.

| Tomato Sauce | | | |
|---|---|---|---|
| | | **Reference** | **HWGC** |
| Sauce | | **%** | **%** |
| | Water | 48.00 | 40.35 |
| | Tomatoes in Juice | 38.90 | 33.00 |
| | Corn starch | 1.20 | 1.00 |
| | Black pepper | 0.06 | 0.05 |
| | Sugar | 1.10 | 0.55 |
| | Salt | 0.70 | 0.60 |
| | Seasoning (garlic, oregano, bay leaves, thyme, basil) | 1.44 | 1.20 |
| | Onion diced 1/4 | 8.60 | 7.25 |
| | HWGC (50% moisture) | 0.00 | 16.00 |
| Subtotal | | 100.00 | 100.00 |
| | | | |
| Final Product | | Grams | Grams |
| | Sauce | 150 | 150 |
| | Pasta | 100 | 100 |
| | | | |
| Final Product Total | | 250 | 250 |

### Example 7 - Chicken with Vegetables and Peanut Sauce

The hydrolyzed whole grain composition (HWGC) is prepared according to example 1. Similar to example 6, this recipe relates both to a food ingredient comprising HWGC and a composite food product comprising HWGC.

| Sauce | | **Reference** | **With HWGC** |
|---|---|---|---|
| | **Ingredients** | **%** | **%** |
| | Milk | 63.00 | 65.50 |
| | Sugar (Sugar, brown sugar) | 8.50 | 6.00 |
| | Peanut Butter | 6.50 | 6.50 |
| | Vinegar | 3.00 | 3.00 |
| | Soy Sauce | 6.00 | 6.00 |
| | Spices (red pepper, black pepper, chili peppers) | 3.00 | 3.00 |
| | Puree (garlic, tamarind) | 4.00 | 4.00 |
| | Coconut milk | 4.00 | 4.00 |
| | Corn starch | 1.50 | 1.50 |
| | Salt | 0.50 | 0.50 |
| | Total | 100.00 | 100.00 |

| Sauce Mix | | | |
|---|---|---|---|
| | **Ingredients** | **%** | **%** |
| | Sauce | 100.00 | 84.00 |
| | HWGC (50% moisture) | 0.00 | 16.00 |
| | Total | 100.00 | 100.00 |

| Vegetable Mix | | | |
|---|---|---|---|
| | **Ingredients** | **%** | **%** |
| | green beans | 45.00 | 45.00 |
| | carrots | 40.00 | 40.00 |
| | bell pepper | 15.00 | 15.00 |
| | Total | 100.00 | 100.00 |

| Final Product | | | |
|---|---|---|---|
| | **Ingredients** | **Grams** | **Grams** |
| | Sauce Mix | 100.00 | 100.00 |
| | Vegetable Mix | 70.00 | 70.00 |
| | Chicken | 55.00 | 55.00 |
| | Pasta (cooked) | 75.00 | 75.00 |
| | Total | 300.00 | 300.00 |

## Claims

1. A food ingredient comprising:
- a savoury flavour component;
- a hydrolyzed whole grain composition; and
- an alpha-amylase or fragment thereof, which alpha-amylase or fragment thereof shows no hydrolytic activity towards dietary fibers when in the active state;
wherein the hydrolyzed whole grain composition has a substantially intact beta-glucan structure relative to the starting material and/or a substantially intact arabinoxylan structure relative to the starting material.

2. The food ingredient according to claim 1, wherein the food ingredient is a sauce, a soup, a pizza topping, a sandwich topping, a sandwich filling, or a dough filling.

3. The food ingredient according to claim 1 or claim 2, wherein the savoury flavour component is a spice.

4. The food ingredient according to any one of the preceding claims, wherein the food ingredient has a maltose to glucose ratio below 144:1 by weight of the food ingredient, such as below 120:1, such as below 100:1, such as below 50:1, such as below 30:1, such as below 20:1 or such as below 10:1.

5. The food ingredient according to any one of the preceding claims, with the proviso that it does not comprise a beta-amylase.

6. The food ingredient according to any one of the preceding claims, further comprising a protease or fragment thereof, at a concentration of 0.001-5% by weight of the total whole grain content, which protease or fragment thereof shows no hydrolytic activity towards dietary fibers when in the active state.

7. The food ingredient according to any one of claims 1-5, with the proviso that it does not comprise a protease or fragment thereof.

8. The food ingredient according to any one of the preceding claims, further comprising at least one of an amyloglucosidase or fragment thereof and an glucose isomerase or fragment thereof which amyloglucosidase or glucose isomerase shows no hydrolytic activity towards dietary fibers when in the active state.

9. The food ingredient according to any one of the preceding claims, having a total content of whole grain in the range of 1-35% by weight of the food ingredient.

10. The food ingredient according to any one of the preceding claims, further comprising a texturing agent, preferably in the range 0.01% to 10% by weight of the food ingredient.

11. A process for preparing the food ingredient according to any one of claims 1-10, said process comprising:
1) preparing a hydrolyzed whole grain composition, comprising the steps of:
a) contacting a whole grain component with an enzyme composition in water said enzyme composition showing no hydrolytic activity towards dietary fibers,
b) allowing the enzyme composition to react with the whole grain component, to provide a whole grain hydrolysate,
c) providing the hydrolyzed whole grain composition by deactivating said enzymes when said hydrolysate has reached a viscosity comprised between 50 and 12000 mPa.s measured at 65°C,
2) providing the food ingredient by mixing the hydrolyzed whole grain composition with a premix comprising a savoury flavour component.

12. The process according to claim 11, wherein the hydrolyzed whole grain composition in step 1) is provided when said hydrolysate has reached a total solid content of 25-50% by weight of the hydrolyzed whole grain composition.

13. A composite food product comprising a food ingredient according to any one of claims 1-10.

## Patentansprüche

1. Nahrungsmittelzutat aufweisend:
- eine geschmackshafte Aromakomponente;
- eine hydrolysierten Vollkornzusammensetzung; und
- eine Alpha-Amylase oder einem Fragment davon, wobei die Alpha-Amylase oder das Fragment davon keine hydrolytische Aktivität gegenüber diätetischen Fibern zeigt, wenn sie/es sich im aktiven Zustand befindet;
wobei die hydrolysierte Vollkornzusammensetzung gegenüber dem Ausgangsmaterial eine im wesentlichen intakte Beta-Glucan-Struktur aufweist und/oder gegenüber dem Ausgangsmaterial eine im Wesentlichen intakte Arabinoxylan-Struktur aufweist.

2. Nahrungsmittelzutat nach Anspruch 1, wobei die Nahrungsmittelzutat eine Soße, eine Suppe, ein Pizzabelag, ein Sandwichbelag, eine Sandwichfüllung oder eine Teigfüllung ist.

3. Nahrungsmittelzutat nach Anspruch 1 oder Anspruch 2, wobei die geschmackshafte Aromakomponente ein Gewürz ist.

4. Nahrungsmittelzutat nach einem der vorhergehenden Ansprüche, wobei die Nahrungsmittelzutat ein Verhältnis von Maltose zu Glucose von unter 144:1 nach Gewicht der Nahrungsmittelzutat besitzt, wie z.B. unter 120:1, wie z.B. unter 100:1, wie z.B. unter 50:1, wie z.B. unter 30:1, wie z.B. unter 20:1 oder wie z.B. unter 10:1.

5. Nahrungsmittelzutat nach einem der vorhergehenden Ansprüche, mit der Maßgabe, dass sie keine Beta-Amylase aufweist.

6. Nahrungsmittelzutat nach einem der vorhergehenden Ansprüche, weiterhin aufweisend eine Protease oder ein Fragment davon, bei einer Konzentration von 0,001-5 Gew.-% des Gesamtvollkorngehaltes, wobei die Protease oder das Fragment davon keine hydrolytische Aktivität gegenüber diätetischen Fibern zeigt, wenn sie/es sich im aktiven Zustand befindet.

7. Nahrungsmittelzutat nach einem der Ansprüche 1-5, mit der Maßgabe, dass sie keine Protease oder ein Fragment davon aufweist.

8. Nahrungsmittelzutat nach einem der vorhergehenden Ansprüche, weiterhin aufweisend mindestens eines aus einer Amyloglucosidase oder einem Fragment davon und einer Glucose-Isomerase oder einem Fragment davon, wobei die Amyloglucosidase oder die Glucose-Isomerase keine hydrolytische Aktivität gegenüber diätetischen Fibern zeigt, wenn sie sich im aktiven Zustand befindet.

9. Nahrungsmittelzutat nach einem der vorhergehenden Ansprüche, die einen Gesamtvollkorngehalt in dem Bereich von 1-35 Gew.-% der Nahrungsmittelzutat aufweist.

10. Nahrungsmittelzutat nach einem der vorhergehenden Ansprüche, weiterhin aufweisend ein Strukturierungsmittel, vorzugsweise in dem Bereich von 0,01 bis 10 Gew.-% der Nahrungsmittelzutat.

11. Verfahren zur Herstellung der Nahrungsmittelzutat nach einem der Ansprüche 1-10, wobei das Verfahren folgendes aufweist:
1) Herstellen einer hydrolysierten Vollkornzusammensetzung mit den folgenden Schritten:
a) in Kontakt bringen einer Vollkornkomponente mit einer Enzymzusammensetzung in Wasser, wobei die Enzymzusammensetzung keine hydrolytische Aktivität gegenüber diätetischen Fibern zeigt,
b) Zulassen, dass die Enzymzusammensetzung mit der Vollkornkomponente reagiert, um ein Vollkornhydrolysat zu liefern,
c) Bereitstellen der hydrolysierten Vollkornzusammensetzung durch Deaktivieren der Enzyme, wenn das Hydrolysat eine Viskosität zwischen 50 und 12000 mPa.s, gemessen bei 65°C, erreicht hat;
2) Bereitstellen der Nahrungsmittelzutat durch Mischen der hydrolysierten Vollkornzusammensetzung mit einem Vorgemisch, das eine geschmackshafte Aromakomponente aufweist.

12. Verfahren nach Anspruch 11, wobei die hydrolysierte Vollkornzusammensetzung in Schritt 1) bereitgestellt ist, wenn das Hydrolysat einen Gesamtfeststoffgehalt von 25-50 Gew.-% der hydrolysierten Vollkornzusammensetzung erreicht hat.

13. Zusammengesetztes Nahrungsmittelprodukt mit einer Nahrungsmittelzutat nach einem der Ansprüche 1-10.

## Revendications

1. Ingrédient alimentaire comprenant :
- un composant de saveur salée;
- une composition de céréales complètes hydrolysée; et
- une alpha-amylase ou un fragment de celle-ci, laquelle alpha-amylase ou lequel fragment de celle-ci ne montre aucune activité hydrolytique envers des fibres alimentaires à l'état actif;
dans lequel la composition de céréales complètes hydrolysée présente une structure de bêta-glucane sensiblement intacte par rapport à la matière de départ et/ou une structure d'arabinoxylane sensiblement intacte par rapport à la matière de départ.

2. Ingrédient alimentaire selon la revendication 1, dans lequel l'ingrédient alimentaire est une sauce, une soupe, une garniture de pizza, une garniture de sandwich, un remplissage de sandwich, ou un remplissage de pâte.

3. Ingrédient alimentaire selon la revendication 1 ou la revendication 2, dans lequel le composant de saveur salée est une épice.

4. Ingrédient alimentaire selon l'une quelconque des revendications précédentes, dans lequel l'ingrédient alimentaire présente un rapport de maltose sur glucose en-dessous de 144:1 en poids de l'ingrédient alimentaire, comme par exemple en-dessous de 120:1, comme par exemple en-dessous de 100:1, comme par exemple en-dessous de 50:1, comme par exemple en-dessous de 30:1, comme par exemple en-dessous de 20:1 ou comme par exemple en-dessous de 10:1.

5. Ingrédient alimentaire selon l'une quelconque des revendications précédentes, avec la restriction qu'il ne comprend pas de bêta-amylase.

6. Ingrédient alimentaire selon l'une quelconque des revendications précédentes, comprenant en outre une protéase ou un fragment de celle-ci, à une concentration de 0,001-5% en poids de la teneur totale en céréales complètes, laquelle protease ou lequel fragment de celle-ci ne montre aucune activité hydrolytique envers des fibres alimentaires à l'état actif.

7. Ingrédient alimentaire selon l'une quelconque des revendications 1-5, avec la restriction qu'il ne comprend pas de protéase ou de fragment de celle-ci.

8. Ingrédient alimentaire selon l'une quelconque des revendications précédentes, comprenant en outre au moins un élément parmi une amyloglucosidase ou un fragment de celle-ci et une glucose isomérase ou un fragment de celle-ci, laquelle amyloglucosidase ou glucose isomérase ne montre aucune activité hydrolytique envers des fibres alimentaires à l'état actif.

9. Ingrédient alimentaire selon l'une quelconque des revendications précédentes, présentant une teneur totale en céréales complètes dans la gamme de 1-35% en poids de l'ingrédient alimentaire.

10. Ingrédient alimentaire selon l'une quelconque des revendications précédentes, comprenant en outre un agent de texturation, de préférence dans la gamme de 0,01% à 10% en poids de l'ingrédient alimentaire.

11. Procédé de préparation de l'ingrédient alimentaire selon l'une quelconque des revendications 1-10, ledit procédé comprenant :
1) la préparation d'une composition de céréales complètes hydrolysée, comprenant les étapes consistant à :
a) mettre en contact un composant de céréales complètes avec une composition d'enzymes dans de l'eau, ladite composition d'enzymes ne montrant aucune activité hydrolytique envers des fibres alimentaires,
b) permettre à la composition d'enzymes de réagir avec le composant de céréales complètes, afin de fournir un hydrolysat de céréales complètes,
c) fournir la composition de céréales complètes hydrolysée en désactivant lesdits enzymes lorsque ledit hydrolysat a atteint une viscosité comprise entre 50 et 12000 mPa.s mesurée à 65°C,
2) la fourniture de l'ingrédient alimentaire en mélangeant la composition de céréales complètes hydrolysée avec un pré-mélange comprenant un composant de saveur salée.

12. Procédé selon la revendication 11, dans lequel la composition de céréales complètes hydrolysée, dans l'étape 1), est fournie lorsque ledit hydrolysat a atteint une teneur totale en matières solides de 25-50% en poids de la composition de céréales complètes hydrolysée.

13. Produit alimentaire composite comprenant un ingrédient alimentaire selon l'une quelconque des revendications 1-10.
